# EUROPÄISCHE PATENTSCHRIFT

(11) **EP 3 281 610 B1**
(45) Veröffentlichungstag und Bekanntmachung des Hinweises auf die Patenterteilung: **22.07.2020**
(21) Anmeldenummer: 17184729.6
(22) Anmeldetag: 03.08.2017
(51) Int. Cl.: A61F 5/01, A61F 5/058

(54) **QUENGELSCHIENE**
ORTHOSIS
ORTHÈSE

(30) Priorität: 10.08.2016 DE 202016104405 U
(43) Veröffentlichungstag der Anmeldung: 14.02.2018
(73) Patentinhaber: Wagner, Helmut, 37115 Duderstadt (DE)
(72) Erfinder: Wagner, Helmut, 37115 Duderstadt (DE)
(74) Vertreter: REHBERG HÜPPE + PARTNER

(56) Entgegenhaltungen:
- EP-A1- 2 959 869
- WO-A1-2016/210121
- DE-B4- 19 904 554
- US-A- 5 575 764
- US-A1- 2002 094 919
- US-A1- 2009 198 161
- US-A1- 2012 071 803
- US-A1- 2014 276 304
- US-A1- 2015 290 010

## Beschreibung

### TECHNISCHES GEBIET DER ERFINDUNG

Die Erfindung betrifft eine Quengelschiene. Eine Quengelschiene ist eine dynamische Gelenkschiene, welche auch als dynamische Redressionsorthese oder dynamisches Korrektur-Gelenksystem oder als Korrektur-Gelenksystem bezeichnet werden kann. Eingesetzt werden können Quengelschienen für eine Behandlung eines bewegungseingeschränkten Gelenks zwischen Extremitäten eines menschlichen oder tierischen Körpers. Mittels der Quengelschiene kann ein Quengelmoment auf die Extremitäten in Richtung der Bewegungseinschränkung oder eine der Richtung einer Kontraktur entgegenwirkende Spannung ausgeübt werden. Möglich ist auch, dass eine Quengelschiene zur Nachbehandlung einer Sehnenverletzung verwendet wird, wobei in diesem Fall das Quengelmoment der Quengelschiene zu einer Entlastung der operativ behandelten Sehne dient. Die Quengelschiene kann für beliebige Extremitäten und zugeordnete Gelenke eingesetzt werden, insbesondere für ein Handgelenk, ein Fingergelenk, ein Ellbogengelenk, ein Kniegelenk, ein Fußgelenk, ein Hüftgelenk u. ä. Möglich medizinische Indikationen für den Einsatz der Quengelschiene können (ohne Beschränkung auf diese) eine orthetische Versorgung mit eingeschränkter Beweglichkeit, eine Behandlung nach Verletzungen mit Kontrakturen, eine Behandlung nach operativen Eingriffen, eine schrittweise Mobilisierung eines Gelenks, eine Behandlung einer von Verkürzung bedrohten spastischen Muskulatur, ein Einsatz für eine Extension oder eine Flexion, eine Behandlung von Patienten mit Spastiken, um Bewegungen zu fördern und durch Spastik bedingten Fehlstellungen und Kontrakturen entgegenzuwirken, neurologische Indikationen wie eine Zerebralparese, ein Schlaganfall, eine Spinalkanalverletzung, multiple Sklerose, Spina Bifida, Schädel-Hirn-Traumata, Muskeldystrophie, Arthrogryposis, orthopädische Indikationen nach einer totalen Knieprothese, Amputationen, Operationen, Bänderverletzungen oder Frakturen sein. Im Rahmen der Erfindung kann die Quengelschiene auch einen Aufsatz bilden, mit welchem eine herkömmliche Schwenkschiene ergänzt werden kann. Während bei der herkömmlichen Schwenkschiene unter Umständen Schienenteile ausschließlich über ein Gelenk miteinander verbunden sind, aber kein Quengelmoment erzeugt wird, besitzt die den Aufsatz bildende Quengelschiene Schienenteile, die über geeignete Befestigungseinrichtungen (im einfachsten Fall Verschraubungen) mit den Schienenteilen der Schwenkschiene verbunden werden können. Mittels der den Aufsatz bildenden Quengelschiene wird dann das erforderliche Quengelmoment erzeugt.

Quengelschienen der hier vorliegenden Art verfügen über zwei verschwenkbar miteinander verbundene Schienenteile, welche jeweils an einer Extremität so befestigt werden, dass die Schienenteile bei der Verschwenkung der Extremitäten mit verschwenkt werden. Während bei den oben angeführten Indikationen in einer Ausgangsstellung der Quengelschiene grundsätzlich kein Quengelmoment erforderlich ist, soll die Quengelschiene mit einer Annäherung an eine Quengelstellung ein in Richtung der Quengelstellung wirkendes Quengelmoment erzeugen.

Im Folgenden wird zur vereinfachenden Erläuterung als Beispiel auf eine Quengelschiene für einen Ellbogen Bezug genommen, welche nach einer operativen Behandlung einer Sehne, die an der Streckung des Ellbogengelenks beteiligt ist, Bezug genommen, ohne dass die Erfindung hinsichtlich des Einsatzbereiches für einen Ellbogen oder hinsichtlich einer Behandlung einer Sehne nach einem operativen Eingriff beschränkt sein soll: Für das genannte Beispiel ist eine Ausgangsstellung des Ellbogengelenks eine maximale Beugestellung des Unterarms gegenüber dem Oberarm, für welche diese einen Beugewinkel von ca. 140° gegenüber der Streckstellung bilden. Hingegen entspricht die Quengelstellung der Quengelschiene in diesem Fall der Streckstellung, also einem Beugewinkel von 0° des Unterarms gegenüber dem Oberarm. Reicht nach dem operativen Eingriff das über die streckende Sehne aufgebrachte Moment nicht aus, um das Ellbogengelenk in die Streckstellung zu bringen, oder gefährdet die alleinige Streckung mittels der streckenden Sehne das operative Ergebnis, soll die Quengelschiene mit Annäherung an die Streckstellung, also die Quengelstellung, ein Quengelmoment erzeugen, welches in Richtung der Quengelstellung wirkt und somit die Streckung des Ellbogengelenks herbeiführt oder die Streckung durch die streckende Sehne unterstützt.

Bei bekannten Quengelschienen findet zwischen den Schienenteilen eine Torsionsfeder Einsatz, welche so vorgespannt ist, dass diese in der Quengelstellung das erforderliche Quengelmoment erzeugt. Diese Torsionsfeder muss mit der Bewegung aus der Quengelstellung in Richtung der Ausgangsstellung weiter gespannt werden, so dass für die Verschwenkung des Unterarms in Richtung der Beugestellung, also für eine Flexion, die an der Beugung beteiligten Muskeln ein erhöhtes Beugemoment erzeugen müssen, welches je nach Charakteristik der Torsionsfeder mit einer Vergrößerung des Beugewinkels linear oder kurvenförmig ansteigt.

### STAND DER TECHNIK

Quengelschienen sind beispielsweise aus
- der Internet-Seite www.caroli.de/dynamische-gelenkschienen (Datum der Einsichtnahme 28.07.2016),
- der Internet-Seite www.prowalk.de/neurologie-orthopaedie/ultraflex (Datum der Einsichtnahme 28.07.2016),
- der Internet-Seite www.basko.com (vgl. Produkt "MultiMotion", Datum der Einsichtnahme 28.07.2016),
- der Produktbroschüre des Unternehmens Otto Bock HealthCare GmbH mit dem Titel "Neuro-Orthopädie", Kapitel zu sonstigen Komponenten, Armschienen bis Korrektur-Gelenksystemen (Seiten 180-183) sowie
- einem Produkt der Pro Walk GmbH mit der Kennzeichnung "Ultraflex" und "ONE" (eingetragene Marken)
bekannt.

Die Druckschriften DE 87 04 851 U1 sowie DE 84 33 416 U1 beschreiben statische Quengelschienen zum Fixieren eines Schwenkwinkels der Extremitäten.

DE 199 04 554 B4 offenbart den Einsatz einer Quengelschiene und eine Mitläuferschiene, welche auf gegenüberliegenden Seiten eines Ellbogens angeordnet werden und jeweils mit ihren entsprechenden Schienenteilen über ein Befestigungsband am Unterarm und am Oberarm befestigt werden. Die Quengelschiene soll hierbei sowohl ein Quengeln in Flexions- als auch in Extensionsrichtung ermöglichen. Über eine Schneckenwelle ist eine Begrenzung der maximalen Flexion und Extension einstellbar. Exzentrisch von der Schwenkachse der Quengelschiene ist an einer Tragscheibe ein Endbereich einer Koppelstange angelenkt. Der andere Endbereich der Koppelstange ist axial gegen eine Feder verschieblich an dem distalen Schienenteil gelagert. Im Umgebungsbereich der Streckstellung erzeugt die federbeaufschlagte Koppelstange ein Quengelmoment in Richtung der Streckstellung, welches mit zunehmender Annäherung an die Streckstellung kleiner wird. Erfolgt (mit zunehmender Muskelkraft des Trägers der Quengelschiene) ausgehend von der Streckstellung ein Beugen der Quengelschiene, verändert sich die Wirkachse der Kurbelstange, bis für einen mittigen Schwenkwinkel die Wirkachse der Kurbelstange durch die Schwenkachse der Quengelschiene verläuft. Mit weiterer Vergrößerung des Beugewinkels kehrt sich die Richtung des Quengelmoments um. Auch mit Vergrößerung des Beugewinkels und Annäherung an die maximale Beugestellung vergrößert sich der Betrag des Quengelmoments.

DE 201 17 080 U1 offenbart eine Quengelschiene, bei welcher das Quengelmoment einen pneumatischen Stellzylinder aufweist. Die Druckbeaufschlagung des pneumatischen Stellzylinders kann mittels einer elektronischen Steuereinheit beeinflusst werden, womit eine elektronische Steuerung des Quengelmoments möglich ist.

Die nicht gattungsgemäße Druckschrift US 2002/0094919 A1 offenbart eine Unterstützungseinrichtung für eine Orthese, Prothese oder einen Roboter, mittels welcher passiv infolge einer Federunterstützung mit verringertem Energieaufwand ein schnelleres Laufen ermöglicht werden soll. Eine Ausführungsform der Unterstützungseinrichtung erstreckt sich dabei von dem Becken oder der Hüfte bis zum Fuß der zu unterstützenden Person oder des zu unterstützenden Roboters. Die Unterstützungseinrichtung verfügt dabei über ein im Bereich des Beckens angeordnetes Geschirr oder einen Leibgurt, sich hiervon verschwenkbar nach unten erstreckende und über ein Kniegelenk miteinander verbundene Stützstreben, einen auf die Stützstreben einwirkenden Federmechanismus und ein an dem unteren Ende der unteren Stützstrebe gehaltenes Fußteil, welches an dem Fuß der Person oder des Roboters befestigt werden kann. Für eine Ausführungsform verfügt die Unterstützungseinrichtung über einen Kniegelenk-Strecker, welcher mit einer Kreisfeder lediglich eine kleine Kraft jenseits eines vorgegebenen Beugewinkels erzeugen soll, so dass eine freie Streckung möglich sein soll. Zwischen den Stützstreben wirkt unmittelbar eine Streckungsfeder. Jenseits des vorgegebenen Beugewinkels verläuft die Wirkachse der Streckungsfeder durch das Kniegelenk, so dass diese die Kraftverhältnisse für die Beugung der Stützstreben nicht beeinflusst. Zwischen dem vorgegebenen Beugungswinkel und der Streckung der Stützstreben hat die Wirkachse der Streckungsfeder aber eine Exzentrizität hinsichtlich des Kniegelenks, womit die Streckungsfeder mit der Annäherung an die Streckung der Stützstreben ein sich vergrößerndes, in Richtung der Streckung wirkendes Moment erzeugt.

EP 2 959 869 A1 offenbart eine orthopädische Laufunterstützung, welche im oberen Endbereich über einen Gurt an der Hüfte sowie im unteren Endbereich über einen weiteren Gurt an dem Oberschenkel befestigt wird. Die orthopädische Laufunterstützung verfügt über zwei über ein Kniegelenk miteinander verbundene Stützstreben. Eine Spanneinrichtung übt ein Schwenkmoment auf die Stützstreben aus, dessen Betrag von dem Schwenkwinkel der Stützstreben abhängt, und welches in Richtung eines vorbestimmten Winkels der Stützstreben wirkt. Die Spanneinrichtung weist eine im Inneren einer Stützstrebe angeordnete Spannfeder auf, deren einer Federfußpunkt mit dieser Stützstrebe verbunden ist, während der andere Federfußpunkt der Spannfeder über ein Spannseil mit der anderen Stützstrebe an einem Ort beabstandet von der Schwenkachse verbunden ist. Im Bereich des Gelenks umschlingt das Seil die Mantelfläche eines Nockens, wobei der Umschlingungswinkel von dem Schwenkwinkel der Stützstreben abhängig ist und eine Vergrößerung des Umfangswinkels mit einer Vergrößerung der Vorspannung der Spannfeder verbunden ist und damit mit einer Vergrößerung des von der Spanneinrichtung auf die Spannstreben ausgeübten Rückstellmoments. Für eine erste Ausführungsform vergrößert sich der Umgangswinkel und damit das Rückstellmoment von einer Ausgangsstellung, in welcher die Stützstreben einen Winkel von 90° einnehmen, in eine gestreckte Stellung, in welcher die Stützstreben einen Winkel von 180° einnehmen. Für eine andere Ausführungsform, bei welcher das Spannseil auf der anderen Seite um den Nocken geschlungen ist, verringert sich das Rückstellmoment von einer Ausgangsstellung, in welcher die Stützstreben einen Winkel von 90° einnehmen, in Richtung einer gestreckten Stellung der Stützstreben.

US 2012/0071803 A1 offenbart eine orthopädische Kniegelenkunterstützung, bei welcher ebenfalls zwei Stützstreben über ein Kniegelenk miteinander verbunden sind. Eine Stützstrebe bildet im Bereich des Kniegelenks eine Nockenscheibe aus, an deren Mantelfläche mit von dem Schwenkwinkel der Stützstreben abhängigem Umfangswinkel ein Spannseil abgestützt ist, welches über eine an der anderen Stützstrebe angelenkte Spannfeder gespannt wird. Die Abstützung des Spannseils an der Nockenscheibe hat zur Folge, dass die Spannkraft des Spannseils mit einem von dem Schwenkwinkel abhängigen Hebelarm auf die erstgenannte Stützstrebe wirkt. Andererseits ist die Spannkraft der Spannfeder und damit die mit dem Hebelarm wirkende Spannkraft abhängig von dem Umfangswinkel, im Bereich dessen das Spannseil die Nockenscheibe umschlingt. Es überwiegt der Einfluss der Auslenkung der Spannfeder je nach Schwenkwinkel gegenüber der Veränderung des Hebelarms je nach Schwenkwinkel. Somit ist ein in Richtung der gestreckten Stellung der Stützstreben orientiertes Unterstützungsmoment der Unterstützungseinrichtung am größten für den maximalen Beugewinkel der Stützstreben, und dieses nimmt in Richtung der gestreckten Stellung kontinuierlich ab.

US 2009/0198161 A1 offenbart eine orthopädische Unterstützungseinrichtung, bei welcher die beiden Stützstreben über ein Verbindungsstück so mit gekoppelt sind, dass diese unabhängig von dem relativen Schwenkwinkel der Stützstreben jeweils mit dem halben Schwenkwinkel gegenüber dem Verbindungsstück abgewinkelt sind. An dem Verbindungsstück ist eine Blattfeder gehalten, an welcher ein an einer Stützstrebe gehaltener Anschlag mit Annäherung an die gestreckte Stellung der Stützstreben zur Anlage kommt. Die Durchbiegung der Blattfeder infolge der Anpressung des Anschlags nimmt in Richtung der gestreckten Stellung der Stützstreben kontinuierlich zu, womit ein sich in Richtung der gestreckten Stellung vergrößerndes Rückstellmoment erzeugt wird, welches darauf ausgerichtet ist, die Stützstreben wieder aus der gestreckten Stellung zurück zu verschwenken.

US 2014/0276304 A1 offenbart eine Abstützeinrichtung, bei der eine Abstützung einer Axiallast mit einer Steifigkeit erfolgt, welche über einen Gleichstrommotor verstellt werden kann. Findet die Abstützeinrichtung Einsatz für eine Kniegelenkorthese, erzeugt diese ein Rückstellmoment, welches in Richtung der gestreckten Lage der Stützstreben und damit des Schienbeins und des Unterschenkels orientiert ist und mit zunehmender Beugung größer wird. Hierbei kann mit einer Vergrößerung des Beugewinkels eine Zuschaltung einer zusätzlichen Feder erfolgen, womit eine nicht lineare Charakteristik des Rückstellmoments in Abhängigkeit von dem Beugewinkel erzeugt werden kann.

Die nicht vorveröffentlichte Druckschrift WO 2016/210121 A1 offenbart eine Kniegelenkorthese, bei welcher eine obere, einem Oberschenkel zugeordnete Stützstrebe im unteren Endbereich gekröpft ist. Im Bereich der Kröpfung ist an der oberen Stützstrebe über ein Kniegelenk eine untere, einem Unterschenkel zugeordnete Stützstrebe angelenkt. An einem unteren, infolge der Kröpfung abgewinkelten Endbereich der oberen Stützstrebe ist verschwenkbar eine Spanneinrichtung angelenkt, in welcher ein Stößel hinsichtlich einer Druckbeanspruchung elastisch gegenüber einem Gehäuse abgestützt ist. Das Gehäuse und der Stößel können hierbei als Luftfeder ausgebildet sein, oder der Stößel kann über eine Druckfeder gegenüber dem Gehäuse abgestützt sein. Der freie Endbereich des Stößels ist über eine schaltbare Kopplungseinrichtung beabstandet von dem Kniegelenk mit der unteren Stützstrebe verbunden. Die Kniegelenkorthese verfügt über drei unterschiedliche Betriebsbereiche: Ein erster Betriebsbereich erstreckt sich von der Strecklage der Stützstreben bis zu einem Umschaltwinkel. Ein zweiter Betriebsbereich erstreckt sich von dem Umschaltwinkel zu einem Freigabewinkel. Schließlich erstreckt sich ein dritter Betriebsbereich von dem Freigabewinkel bis zu einem maximalen Beugewinkel. Der Umschaltwinkel, für welchen der Übergang von dem ersten Betriebsbereich zu dem zweiten Betriebsbereich erfolgt, ist dadurch vorgegeben, dass die Wirkachse der Spanneinrichtung durch das Kniegelenk verläuft, womit die Spanneinrichtung kein Moment auf die Stützstreben ausüben kann. In dem ersten Betriebsbereich erzeugt die Spanneinrichtung ein Moment hinsichtlich der beiden Stützstreben, welches in Richtung der Strecklage orientiert ist, in der Strecklage Null beträgt, dann mit einer Vergrößerung des Beugewinkels in dem ersten Betriebsbereich zunächst ansteigt und mit Annäherung an den Umschaltwinkel wieder auf Null abfällt. Hingegen erzeugt die Spanneinrichtung in dem zweiten Betriebsbereich ein auf die Stützstreben wirkendes Moment, welches in Richtung einer Vergrößerung des Beugewinkels orientiert ist. Dieses Moment steigt mit Vergrößerung des Beugewinkels zunächst von dem Umschaltwinkel in dem zweiten Betriebsbereich an, um dann mit dem Erreichen des Freigabewinkels wieder auf Null abzufallen. Mit dem Übergang zu dem dritten Betriebsbereich, also dem Erreichen des Freigabewinkels, hat die Spanneinrichtung ihre entspannte Auslenkung erreicht. Erfolgt in dem dritten Betriebsbereich eine Vergrößerung des Beugewinkels über den Freigabewinkel hinaus, erfolgt über die Kopplungseinrichtung eine Freigabe der Verbindung zwischen der Spanneinrichtung und der unteren Stützstrebe, womit die Spanneinrichtung kein Moment mehr auf die Stützstreben ausüben kann. Wird für eine Rückbewegung der Stützstreben in Streckungsrichtung hingegen der Freigabewinkel wieder erreicht, führt die Kopplungseinrichtung automatisch wieder eine Kopplung der Spanneinrichtung mit der unteren Stützstrebe herbei.

### AUFGABE DER ERFINDUNG

Der Erfindung liegt die Aufgabe zugrunde, eine dynamische Quengelschiene vorzuschlagen, die eine alternative oder verbesserte Charakteristik des Quengelmoments in Abhängigkeit von dem Schwenkwinkel der Quengelschiene aufweist, was insbesondere mit einem einfachen konstruktiven Aufbau gewährleistet werden soll.

### LÖSUNG

Die Aufgabe der Erfindung wird erfindungsgemäß mit den Merkmalen des unabhängigen Patentanspruchs gelöst. Weitere bevorzugte erfindungsgemäße Ausgestaltungen sind den abhängigen Patentansprüchen zu entnehmen.

### BESCHREIBUNG DER ERFINDUNG

Die erfindungsgemäße dynamische Quengelschiene weist zwei Schienenteile und eine (grundsätzlich beliebig aufgebaute) Federeinrichtung auf. Die Federfußpunkte der Federeinrichtung sind jeweils mit einem der Schienenteile gekoppelt. Die Kopplung erfolgt derart, dass eine Verschwenkung der Schienenteile zu einer veränderten Beaufschlagung der Federeinrichtung führt.

Vorzugsweise ist die Federeinrichtung zumindest in einem Schwenkbereich (unmittelbar oder mittelbar) an den beiden Schienenteilen angelenkt, so dass die Bewegung eines Federfußpunktes der Bewegung des Schienenteils entspricht. Die Federeinrichtung übt ein in Richtung einer Quengelstellung der Schienenteile wirkendes Quengelmoment auf die Schienenteile aus. Hierbei ist die Quengelstellung eine Stellung mit maximaler Extension.

Erfindungsgemäß wird vorgeschlagen, die Federeinrichtung so auszubilden und so mit den Schienenteilen zu koppeln, dass der Betrag des Quengelmoments mit Annäherung an die Quengelstellung der Schienenteile größer wird. Anders gesagt ist erfindungsgemäß das Quengelmoment in der Quengelstellung größer als in einer der Quengelstellung (unmittelbar) benachbarten Schwenkstellung der Schienenteile. Vorzugsweise ist das Quengelmoment in der Quengelstellung größer als für sämtliche möglichen anderen Schwenkwinkel der Schienenteile. In der Quengelstellung weist die Charakteristik des Betrags des Quengelmoments ein absolutes oder relatives Maximum auf.

Infolge der erfindungsgemäßen Ausgestaltung ergibt sich eine Charakteristik des Quengelmoments, die zur Folge hat, dass für eine Bewegung der Quengelschiene von der Quengelstellung weg der Benutzer kleiner werdende Muskelkräfte aufbringen muss, womit der Tragekomfort verbessert wird. U. U. kann auch die therapeutische Wirkung der Quengelschiene verbessert werden, wenn das Quengelmoment mit Annäherung an die Quengelstellung größer wird.

In der erfindungsgemäßen Quengelschiene können die Schienenteile unmittelbar die Schienen ausbilden, welche sich parallel zu den Extremitäten der die Quengelschiene tragenden Person erstrecken und endseitig mit diesen verbunden sind. Möglich ist aber auch, dass die Schienenteile die Teile der Quengelschiene sind, an denen die sich parallel zu den Extremitäten der die Quengelschiene tragenden Person erstreckenden und endseitig mit diesen verbundenen Schienen befestigt werden. In diesem Fall oder auch im Fall der Ausbildung der Quengelschiene als Aufsatz für eine Schwenkschiene bildet die erfindungsgemäße Quengelschiene eine Teil-Baueinheit, welche mit anderen Teilen wie den vorgenannten Schienen oder Teil-Baueinheiten wie der Schwenkschiene zu einer Orthese montiert werden kann.

Erfindungsgemäß wird des Weiteren vorgeschlagen, dass die Quengelschiene einen Umschaltmechanismus aufweist. Dieser Umschaltmechanismus wird bewegungsgesteuert durch die Verschwenkung der Schienenteile betätigt. Infolge der Betätigung des Umschaltmechanismus verändert sich die Kopplung der Schienenteile mit der Federeinrichtung, womit eine Beeinflussung der Charakteristik des Quengelmoments erfolgt. Möglich ist beispielsweise auch, dass in einem Betriebszustand der Umschalteinrichtung die Federeinrichtung ein in Richtung der Quengelstellung wirkendes und auf die Schienenteile ausgeübtes Quengelmoment erzeugt, dessen Betrag von dem Schwenkwinkel der Schienenteile abhängig ist und welches mit Annäherung an die Quengelstellung größer wird, während in dem anderen Betriebszustand der Umschalteinrichtung die Federeinrichtung ein ebenfalls in Richtung der Quengelstellung wirkendes und auf die Schienenteile ausgeübtes Quengelmoment, dessen Betrag unabhängig von dem Schwenkwinkel der Schienenteile konstant ist, oder sogar gar kein Quengelmoment erzeugt.

Bei der Quengelstellung handelt es sich erfindungsgemäß um eine Stellung der Schienenteile mit maximaler Extension, wobei u. U. auch eine Über-Extension möglich ist. Somit wirkt das Quengelmoment in Richtung einer Flexion. Für einen Vorschlag der Erfindung ist die Quengelstellung die Streckstellung der Schienenteile, während alternativ oder kumulativ eine Ausgangsstellung der Quengelschiene eine maximale Beugestellung der Schienenteile mit maximalem Beugewinkel der Schienenteile ist.

Erfindungsgemäß weist die Quengelschiene einen Anschlag auf, über den die Quengelstellung vorgegeben ist, wobei ergänzend auch möglich ist, dass auch die Ausgangsstellung über einen Anschlag vorgegeben ist. Hierbei kann der Anschlag einem festen Schwenkwinkel zugeordnet sein. Möglich ist aber auch, dass der Anschlag einstellbar ist, womit eine Einstellung der Quengelstellung und/oder der Ausgangsstellung möglich ist.

Erfindungsgemäß ist in der Quengelschiene als Federeinrichtung eine Längsfeder genutzt. Hierbei handelt es sich um eine mit einer Normal- oder Längskraft beanspruchte Feder, beispielsweise eine Zugfeder, eine Druckfeder, ein elastisches Band u. ä. In einer derartigen Längsfeder ist die Federkraft von einer Dehnung derselben in Längsrichtung abhängig. Erfindungsgemäß besteht der Umschaltmechanismus darin, dass in einem ersten Schwenkbereich die Längsfeder ausschließlich über die Federfußpunkte der Längsfeder mit den Schienenteilen gekoppelt ist, wozu vorzugsweise die Federfußpunkte unmittelbar an einem Schienenteil oder einem hieran befestigten Tragkörper angelenkt sind. Hingegen ist die Längsfeder in einem zweiten Schwenkbereich zusätzlich zwischen den beiden Federfußpunkten an einer Abstützung abgestützt. Hierbei ist die Abstützung vorzugsweise von einem der Schienenteile getragen oder ausgebildet. Im Bereich der Abstützung erfolgt eine Umlenkung der Längsfeder, womit sich die Kopplung der Federeinrichtung mit den Schienenteilen ändert und sich die Federcharakteristik in Abhängigkeit von dem Schwenkwinkel der Schienenteile verändert. Die Betätigung des Umschaltmechanismus erfolgt durch die Ausbildung des Kontakts der Längsfeder mit der Abstützung, was bewegungsgesteuert mit der Bewegung der Schienenteile erfolgt.

Vorzugsweise beaufschlagt in der Quengelstellung und/oder in der Ausgangsstellung die Federeinrichtung ein Schienenteil gegen den Anschlag, welcher beispielsweise von dem anderen Schienenteil ausgebildet oder getragen sein kann.

Eine Beeinflussung der Charakteristik des Quengelmoments kann auf vielfältige Weise erfolgen. So kann beispielsweise über die Wahl der Federsteifigkeit und/oder eine Nichtlinearität der Federsteifigkeit in Abhängigkeit von dem Schwenkwinkel eine Beeinflussung der Charakteristik des Quengelmoments erfolgen. Möglich ist auch, dass Federfußpunkte der Federeinrichtung an den Schienenteilen der Quengelschiene so angeordnet sind, dass die Änderung des Abstands der Federfußpunkte in nichtlinearer Weise abhängt von dem Schwenkwinkel der Schienenteile. Ebenfalls möglich ist, dass die Federfußpunkte nicht fest an den Schienenteilen gehalten sind, sondern an einem Tragkörper gehalten sind, dessen relative Lage oder Winkel gegenüber dem Schienenteil sich mit der Verschwenkung der Schienenteile ändert.

Für eine Weiterbildung der erfindungsgemäßen Quengelschiene wird vorgeschlagen, dass der erste Schwenkbereich der Quengelstellung benachbart ist, oder dieser die Quengelstellung am Rand einschließt. In diesem ersten Schwenkbereich sind die Schienenteile über eine erste Kopplung mit der Federeinrichtung gekoppelt. Der zweite Schwenkbereich ist der Ausgangsstellung benachbart oder schließt diese im Bereich des Randes ein. In dem zweiten Schwenkbereich sind die Schienenteile über eine zweite Kopplung mit der Federeinrichtung gekoppelt. Die zweite Kopplung und die erste Kopplung unterscheiden sich voneinander. Ein Übergang (der für einen diskreten Schwenkwinkel erfolgen kann oder auch in einem Schwenkwinkelbereich erfolgen kann) von dem ersten Schwenkbereich zu dem zweiten Schwenkbereich (und/oder umgekehrt) erfolgt über die Betätigung des Umschaltmechanismus. Hierbei wird der Umschaltmechanismus bewegungsgesteuert durch die Verschwenkung der Schienenteile betätigt.

Grundsätzlich kann die Charakteristik des Quengelmoments beliebig sein. In besonderer Ausgestaltung der Erfindung wird vorgeschlagen, dass das Quengelmoment in dem zweiten Schwenkbereich konstant ist, Null beträgt und/oder maximal 30 % (vorzugsweise maximal 20 % oder maximal 10 %) des Mittelwerts des Betrags des Quengelmoments im ersten Schwenkbereich beträgt. Dieser Ausgestaltung liegt insbesondere die Erkenntnis zugrunde, dass u. U. die Wirkung des Quengelmoments lediglich im ersten Schwenkbereich, also in der Umgebung der Quengelstellung, gewünscht ist, während ein etwaiges Quengelmoment in dem zweiten Schwenkbereich als "notwendiges Übel" hingenommen werden muss, da der Benutzer für eine Verschwenkung in dem zweiten Schwenkbereich ein Moment aufbringen bzw. überwinden muss. Ist in dem zweiten Schwenkbereich das Quengelmoment konstant, Null oder verhältnismäßig klein, erhöht dies u. U. den Tragekomfort der Quengelschiene.

Grundsätzlich kann die Charakteristik des Quengelmoments, der Federeinrichtung und/oder der Kopplung der Federeinrichtung mit den Schienenteilen (bis auf einen etwaigen Umschaltmechanismus) vom Hersteller fest vorgegeben sein. Bspw. um eine Anpassbarkeit an eine Nutzung der Quengelschiene für unterschiedliche Einsatzorte am menschlichen Körper, für unterschiedliche kinematische Bedingungen und Dimensionen der Extremitäten und des zugeordneten Gelenks und/oder unterschiedliche Verletzungen, operative Eingriffe und Heilungsstufen zu ermöglichen, kann gemäß einem weiteren Vorschlag der Erfindung aber die Charakteristik des Quengelmoments, der Federeinrichtung und/oder der Kopplung der Federeinrichtung mit den Schienenteilen einstellbar sein. Hierbei ist eine Einstellung in Stufen oder auch eine stufenlose Einstellung möglich.

Für eine konstruktive Ausgestaltung der Quengelschiene sind Federfußpunkte der Federeinrichtung jeweils mit einer Exzentrizität gegenüber einer Schwenkachse der Schienenteile mit einem zugeordneten Schienenteil gekoppelt. In dem Teilbereich, in dem der Betrag des Quengelmoments mit zunehmender Annäherung an die Quengelstellung größer wird, sind für diese Ausgestaltung die Schienenteile unter einem Winkel angeordnet, der größer ist als 180°. Gleichzeitig ist der Winkel von Verbindungsachsen der Federfußpunkte der Federeinrichtung mit der Schwenkachse der Quengelschiene kleiner als 180°. Möglich ist hierbei, dass ein an einem Schienenteil fixierter Federfußpunkt auf einer Seite beabstandet von einer Längsachse des anderen Schienenteils angeordnet ist, während sich dann der Grundkörper des erstgenannten Schienenteils auf der anderen Seite der genannten Längsachse erstreckt. Möglich ist, dass der Winkel der Verbindungsachsen der Federfußpunkte mit der Schwenkachse mit zunehmender Annäherung an die Quengelstellung kleiner wird. Dies hat zur Folge, dass sich der Hebelarm der Federeinrichtung vergrößert, womit dann das sich vergrößernde Quengelmoment erzeugt werden kann.

Je nach Ort der Abstützung kann beliebig Einfluss auf die Charakteristik des Quengelmoments genommen werden. Für einen besonderen Vorschlag der Erfindung ist die zusätzliche Abstützung der Längsfeder im Bereich der Schwenkachse der Schienenteile angeordnet. Dies hat zur Folge, dass die Wirkachsen der Teilbereiche der Längsfeder einerseits zwischen einem ersten Federfußpunkt und der Abstützung und andererseits zwischen einem zweiten Federfußpunkt und der Abstützung durch die Schwenkachse verlaufen (oder lediglich mit einem geringen Abstand von dieser verlaufen), so dass die Federkraft der Längsfeder in diesen beiden Teilbereichen keinen (oder einen kleinen) Hebelarm hat. Dies hat zur Folge, dass in dem zweiten Schwenkbereich ein Quengelmoment von Null oder ein entsprechend der Größe des Hebelarms kleines Quengelmoment erzeugt wird, was für den Benutzer eine Verschwenkung in dem zweiten Schwenkbereich vereinfacht.

Grundsätzlich kann die Quengelschiene mit beliebigen Bauelementen in einem beliebigen Fertigungsverfahren hergestellt sein. Möglich ist beispielsweise, dass es sich bei den Schienenteilen um Schmiede- oder Gussteile handelt. Hierbei können beliebige Materialien, insbesondere ein hochfester oder faserverstärkter Kunststoff, Aluminium, Titan oder Stahl, Einsatz finden. Vorzugsweise sind die Schienenteile in einer Plattenbauweise (insbesondere mit metallischen Platten) ausgebildet, wobei die Schienenteile im Bereich des Gelenks der Quengelschiene unmittelbar aneinander anliegen können oder zwischen relativ zueinander bewegten oder verschwenkten Teilen zusätzliche Gleitkörper oder Gleitscheiben, gleitende Beschichtungen oder anderweitige Gleitkörper angeordnet sein können.

Gegenstand der Patentansprüche sind Quengelschienen der zuvor erläuterten Art. Die Patentansprüche umfassen auch die Ausbildung der Quengelschiene als eine Art Aufsatz, der über Schienenteile (welche beliebig geformt sein können und auch nicht als "Schienen" oder lang gestreckte Streben ausgebildet sein müssen) an Schienen einer Gelenkschiene beliebiger Bauart befestigt werden kann. Die sich aus dem von der Quengelschiene gebildeten Aufsatz und der Gelenkschiene zusammensetzende Orthese erzeugt dann das erforderliche Quengelmoment. Hierbei ist möglich, dass eine Verbindung der Schienenteile der Quengelschiene und der Schienenteile der Gelenkschiene in fester, vorgegebener Orientierung erfolgt. Möglich ist aber ebenfalls, dass die Verbindung in unterschiedlichen Winkellagen möglich ist oder einstellbar ist.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Gebrauchsmusters Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einer Feder die Rede ist, ist dies so zu verstehen, dass genau eine Feder, zwei Federn oder mehr Federn vorhanden sind. Diese Merkmale können durch andere Merkmale ergänzt werden oder die einzigen Merkmale sein, aus denen das jeweilige Erzeugnis besteht.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: zeigt eine rückseitige Ansicht einer Quengelschiene in einer Streckstellung.
- **Fig. 2**: zeigt die Quengelschiene gemäß Fig. 1 in einer Seitenansicht.
- **Fig. 3**: zeigt die Quengelschiene gemäß Fig. 1 und 2 in einer Vorderansicht.
- **Fig. 4**: zeigt die Quengelschiene gemäß Fig. 1 bis 3 in einer räumlichen Ansicht schräg von vorne.
- **Fig. 5 bis 10**: zeigen die Quengelschiene gemäß Fig. 1 bis 4 in unterschiedlichen Beugestellungen, wobei Fig. 5, 7, 8, 9, 10 Vorderansichten zeigen, während Fig. 6 eine räumliche Ansicht schräg von vorne zeigt.
- **Fig. 11**: zeigt einen Verlauf eines Hebelarms, einer Federkraft sowie eines Quengelmoments in Abhängigkeit von dem Schwenkwinkel von Schienenteilen der Quengelschiene gemäß Fig. 1 bis 10.
- **Fig. 12 und 13**: zeigen ausgewählte Teilbereiche der Verläufe gemäß Fig. 11 für unterschiedliche Anwendungen, Auslegungen und Einstellungen der Quengelschiene gemäß Fig. 1 bis 10.

### FIGURENBESCHREIBUNG

Die Figuren zeigen eine Quengelschiene 1. Die Quengelschiene 1 kann alleine verwendet werden, wozu diese einseitig über Bänder oder anderweitige Befestigungseinrichtungen an den Extremitäten mit dazwischen angeordnetem Gelenk der zu behandelnden Person befestigt werden kann. Vorzugsweise erfolgt aber ein Anlegen von zwei derartigen Quengelschienen oder einer Quengelschiene und einer Mitläuferschiene auf gegenüberliegenden Seiten der Extremitäten und des Gelenkes mit der Verbindung der beiden Quengelschienen und der Befestigung an den Extremitäten über Befestigungsbänder oder andere Befestigungseinrichtungen (vgl. bspw. DE 199 04 554 B4).

Funktional werden an der Quengelschiene 1 das Gelenk 2 und die über das Gelenk 2 miteinander verbundenen stangenartigen Grundkörper 3, 4 unterschieden. Die Grundkörper 3, 4 erstrecken sich im Gebrauch parallel zu den Extremitäten. Die Grundkörper 3, 4 dienen der Übertragung des Quengelmoments auf die Extremitäten und der Befestigung der Quengelschiene 1 an diesen. Das Gelenk 2 dient der Ermöglichung einer Veränderung des Schwenkwinkels oder Beugewinkels der Quengelschiene 1. In das Gelenk 2 sind weitere Funktionen integriert, insbesondere
- eine Vorgabe des Bereichs der möglichen Schwenkwinkel der Quengelschiene 1 über Anschläge und/oder einen Schneckentrieb,
- die Federeinrichtung zur Erzeugung des Quengelmoments und
- Maßnahmen zur Beeinflussung der Charakteristik des Quengelmoments.

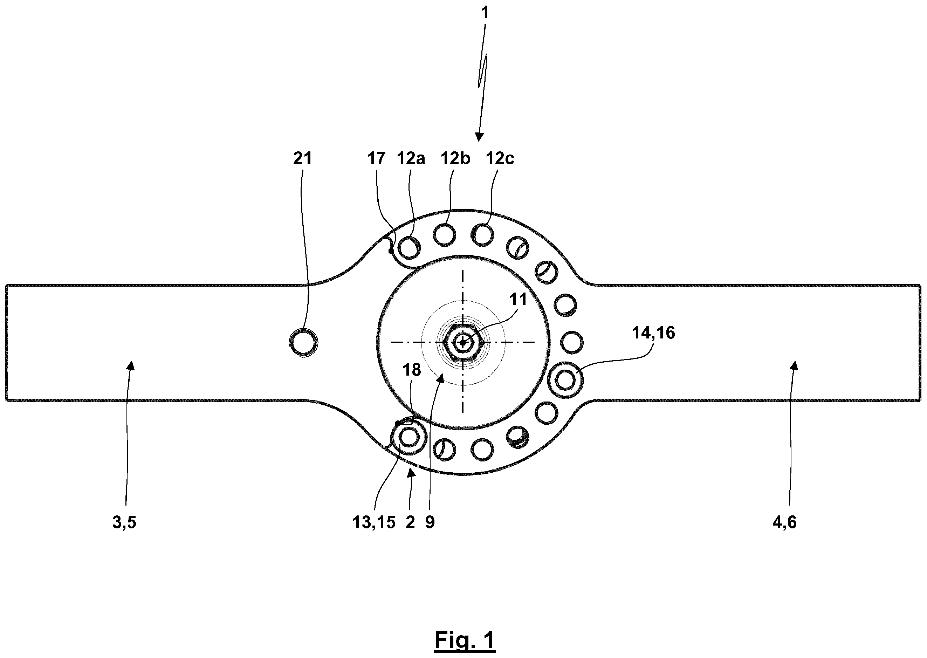

Konstruktiv ist die Quengelschiene 1 mit drei plattenförmigen Bauelementen, nämlich einem Schienenteil 5, einem Schienenteil 6 und einem Tragkörper 7, hier eine Tragscheibe 8, gebildet. Das Schienenteil 6 und das Schienenteil 5 liegen in dieser Reihenfolge im Bereich des Gelenks 2 aneinander an, wobei die Anlage unmittelbar erfolgen kann oder zwischen diesen eine Gleitscheibe angeordnet sein kann, um die Reibung bei einer Verschwenkung zu reduzieren. Die Schienenteile 5, 6 und die Tragscheibe 8 weisen jeweils eine zentrale Bohrung auf, durch welche sich von der Rückseite eine Tellerschraube 9 erstreckt, welche auf der gegenüberliegenden Seite mit einer Abstützung 10 verbunden (insbesondere verschraubt) ist. Die Schienenteile 5, 6 sowie die Tragscheibe 8 und etwaige dazwischen angeordnete Gleitscheiben sind so zwischen dem Kopf der Tellerschraube 9 und der Abstützung 10 gefangen, dass diese eine Verschwenkung um eine Schwenkachse 11 ausführen können, die vertikal zur Zeichenebene gemäß **Fig. 1** verläuft.

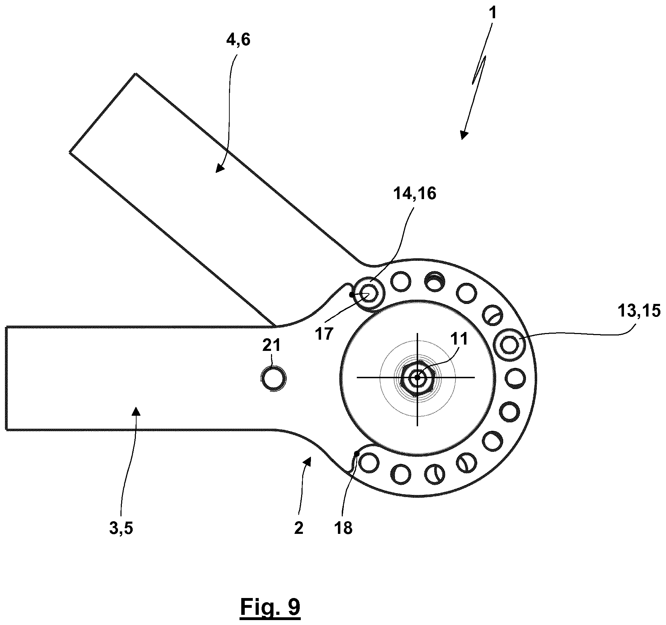

Das Schienenteil 6 weist im Bereich des Gelenks 2 gleichmäßig über den Umfang verteilte Bohrungen 12a, 12b ... auf. In zwei Bohrungen 12 sind zwei Anschläge 13, 14 befestigt. Vorzugsweise handelt es sich bei den Anschlägen 13, 14 um Schrauben 15, 16, die einen Zylinderkopf mit Innensechskant aufweisen und in die dann mit einem Gewinde ausgestatteten Bohrungen 12 eingeschraubt werden können. Die Anschläge 13, 14 dienen jeweils zur Begrenzung des möglichen Schwenkwinkels der Schienenteile 5, 6 in eine Richtung. Um trotz der Anschläge 13, 14 innerhalb des vorgegebenen Schwenkbereichs eine Kollision der Anschläge 13, 14 mit dem Schienenteil 5 zu vermeiden, besitzt das Schienenteil 5 in dem dem Schienenteil 6 zugewandten Teilumfangsbereich einen Radius, der kleiner ist als der Abstand der Anschläge 13, 14 von der Schwenkachse 11. Dieser Teilumfangsbereich ist begrenzt durch beidseitige Gegenanschläge 17, 18, welche von hier abgerundeten radialen Erweiterungen des Schienenteils 5 (in Fig. 1 in einer 11-Uhr-Position sowie einer 7-Uhr-Position hinsichtlich der Schwenkachse 11) gebildet sind. In der in Fig. 1 dargestellten Streckstellung liegt der Anschlag 13 an dem Gegenanschlag 18 an, womit vermieden ist, dass in Fig. 1 bei fixiertem Schienenteil 5 das Schienenteil 6 über die Streckstellung hinaus im Uhrzeigersinn verschwenkt wird. Hingegen ist eine Verschwenkung des Schienenteils 6 gegen den Uhrzeigersinn möglich, bis der Anschlag 14 zu Anlage an den Gegenanschlag 17 kommt, womit der maximale Beugewinkel vorgegeben ist (vgl. Fig. 9). Der minimale und maximale Beugewinkel ist vorgegeben über die Umfangsposition der Anschläge 13, 14 (sowie der Umfangsposition der Gegenanschläge 17, 18). Über die Wahl der Bohrung 12 für die Befestigung der Anschläge 13, 14 kann somit der minimale Beugewinkel, der maximale Beugewinkel und der mögliche Schwenkbereich der Schienenteile 5, 6 vorgegeben werden.

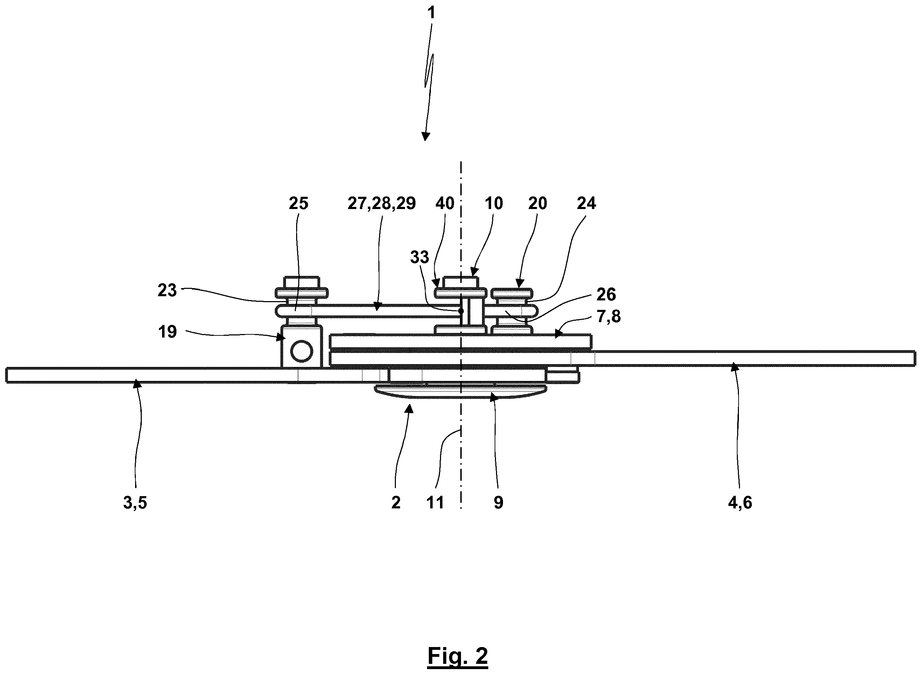

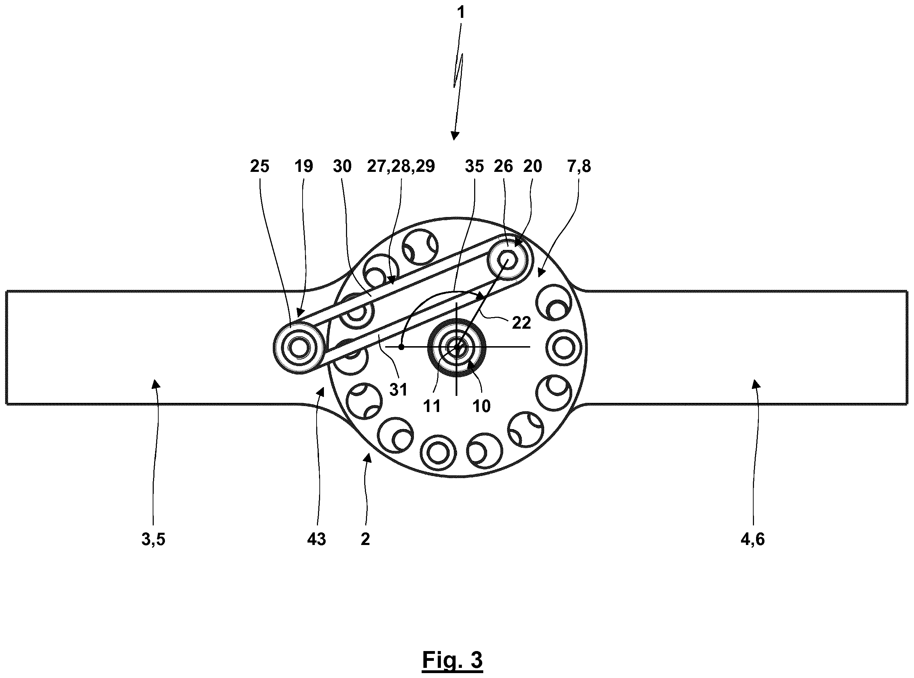

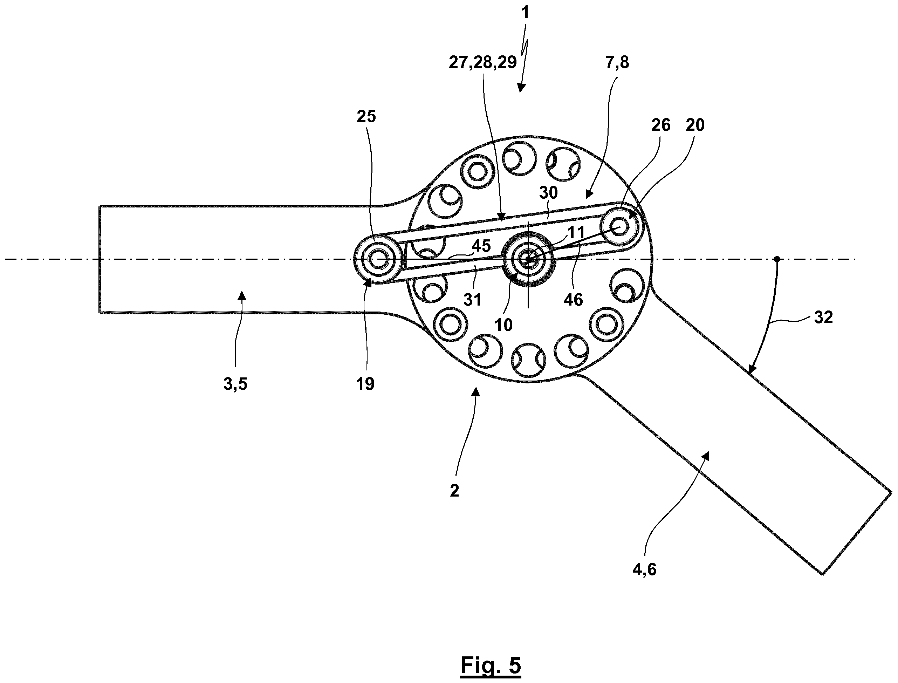

In **Fig. 2** **und** **3** ist zu erkennen, dass an der Tragscheibe 8 und an dem Schienenteil 5 jeweils ein Tragbolzen 19 befestigt ist, was hier beispielsweise durch Einschrauben eines Gewindes der Tragbolzen 19, 20 in entsprechende Gewindebohrungen des Schienenteils 5 und der Tragscheibe 8 erfolgt. Für das dargestellte Ausführungsbeispiel befindet sich die Gewindebohrung 21 für den Tragbolzen 19 auf der Längsachse des Schienenteils 5 im Übergangsbereich von dem Gelenk 2 zu dem Grundkörper 3. In Fig. 3 ist zu erkennen, dass der Tragbolzen 20 mit einer Exzentrizität bzw. einem Radius 22 zu der Schwenkachse 11 an der Tragscheibe 8 gehalten ist. Die Tragbolzen 19, 20 verfügen vorzugsweise über eine Innensechskant-Angriffsfläche, welche das Einschrauben der Tragbolzen 19, 20 in das Schienenteil 5 bzw. die Tragscheibe 8 ermöglicht. Die Tragbolzen 19, 20 besitzen jeweils eine Nut 23, 24. Die Tragbolzen 19, 20 sind parallel zur Schwenkachse 11 angeordnet und erstrecken sich auf der Vorderseite der Quengelschiene 1. Federfußpunkte 25, 26 einer Federeinrichtung 27 sind an den Tragbolzen 19, 20 und hiermit an dem Schienenteil 5 und an der Tragscheibe 8 angelenkt. Für das dargestellte Ausführungsbeispiel ist die Federeinrichtung 27 als Zugfeder 28, hier in Ausbildung als endloses elastisches Band 29, ausgebildet. Das elastische Band 29 ist unter Vorspannung in die Nuten 23, 24 der Tragbolzen 19, 20 eingehängt. Das elastische Band 29 bildet zwischen den Tragbolzen 19, 20 zwei vorgespannte Bandteile 30, 31 aus. Beide Bandteile 30, 31 erzeugen eine Teilfederkraft, welche hinsichtlich der Schwenkachse 11 einen Teilhebelarm aufweisen und somit jeweils ein Teilquengelmoment an der Quengelschiene 1 hervorrufen. Zur Vereinfachung wird im Folgenden von einer von beiden Bandteilen 30, 31 gemeinsam herbeigeführten Federkraft mit einem zugeordneten Federarm und einem gesprochen. Das hieraus resultierende Quengelmoment wirkt in der Streckstellung der Quengelschiene 1 gemäß den Fig. 1 bis 4 derart, dass der Anschlag 13 gegen den Gegenanschlag 18 gezogen wird, womit die Streckstellung aufrechterhalten wird. Ein Verlassen der Streckstellung mit einer Erhöhung des Beugewinkels erfordert, dass ein Schwenkmoment durch den Benutzer auf die Schienenteile 5, 6 ausgeübt wird, um die Kontaktkraft zwischen dem Anschlag 13 und dem Gegenanschlag 18 zu reduzieren und dann den Anschlag 13 von dem Gegenanschlag 18 weg zu bewegen. Diese Beugung aus der Streckstellung gemäß Fig. 3 in eine Beugestellung gemäß Fig. 5 führt dazu, dass sich der Tragbolzen 20 im Uhrzeigersinn um die Schwenkachse 11 bewegt, womit sich der Abstand der Tragbolzen 19, 20 vergrößert und damit die Federkraft der Zugfeder 28 größer wird. Gleichzeitig verringert sich aber der Hebelarm der Zugfeder 28 hinsichtlich der Schwenkachse 11.

Die Tragscheibe 8 ist starr mit dem Schienenteil 6 verbunden. Möglich ist, dass die starre Verbindung zur Einstellung der Quengelschiene 1 in unterschiedlichen Winkelpositionen möglich ist, womit eine Einstellbarkeit gewährleistet ist, der Tragbolzen 20 zwecks Einstellung in unterschiedlichen Umfangspositionen an der Tragscheibe 8 befestigt werden kann und/oder eine stufenlose Einstellung der relativen Lage der Tragscheibe 8 über einen Schneckentrieb möglich ist.

In der Streckstellung gemäß Fig. 1 bis 4 und für kleine Beugewinkel 32 (vgl. Fig. 5) ist die Federeinrichtung 27 lediglich mit den Federfußpunkten 25, 26 an den Tragbolzen 19, 20 bzw. dem Schienenteil 5 und der Tragscheibe 8 angelenkt. Insbesondere besteht keine Wechselwirkung in Form eines Kontakts mit der Abstützung 10. Dies stellt einer erste Kopplung 43 dar.

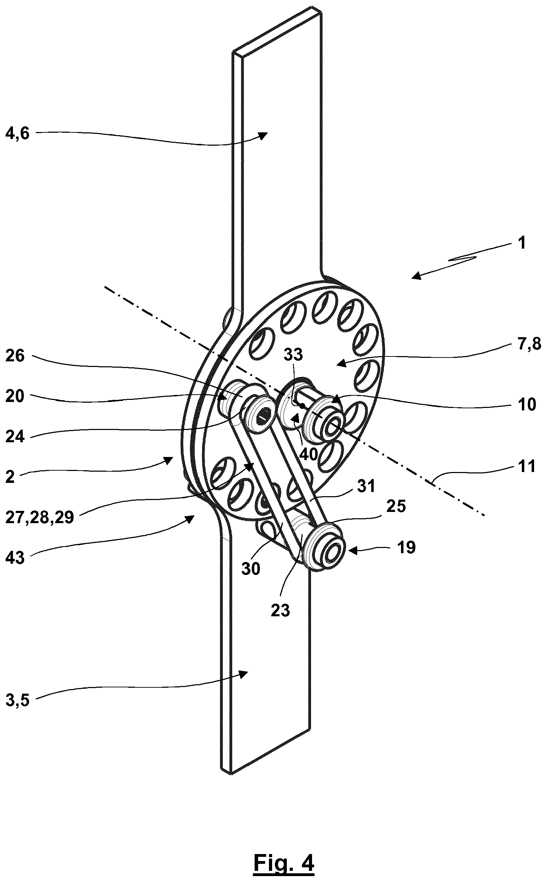

Wie insbesondere in der räumlichen Ansicht gemäß **Fig. 4** zu erkennen ist, ist die Abstützung 10 mit einer Exzentrizität oder einem kurbelartigen Teilbereich in der Bewegungsebene der Federeinrichtung 27 ausgebildet. Hierbei ist die Exzentrizität oder der Versatz des kurbelartigen Teilbereichs derart dimensioniert, dass die Abstützung 10 eine Abstützfläche 33 ausbildet, durch welche sich die Schwenkachse 11 erstreckt. Für den Beugewinkel 32 gemäß **Fig. 5****,** welcher hier ungefähr 45° beträgt, ist das Bandteil 31 bereits zur Anlage an die Abstützfläche 33 gekommen. Die Teilfederkraft des Bandteils 31 verläuft somit durch die Schwenkachse 11, was zur Folge hat, dass das Bandteil 31 keine Teilquengelkraft erzeugt. Allerdings weist das andere Bandteil 30 noch einen kleinen Hebelarm gegenüber der Schwenkachse 11 auf, so dass die Teilfederkraft dieses Bandteils 30 ein (gegenüber dem Quengelmoment in Streckstellung gemäß Fig. 1 bis 4 reduziertes) Quengelmoment erzeugt.

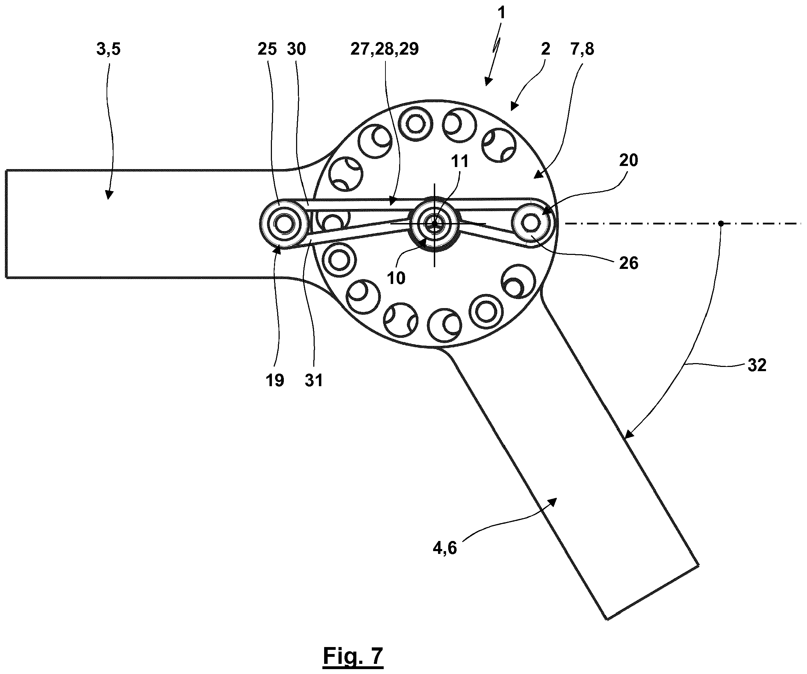

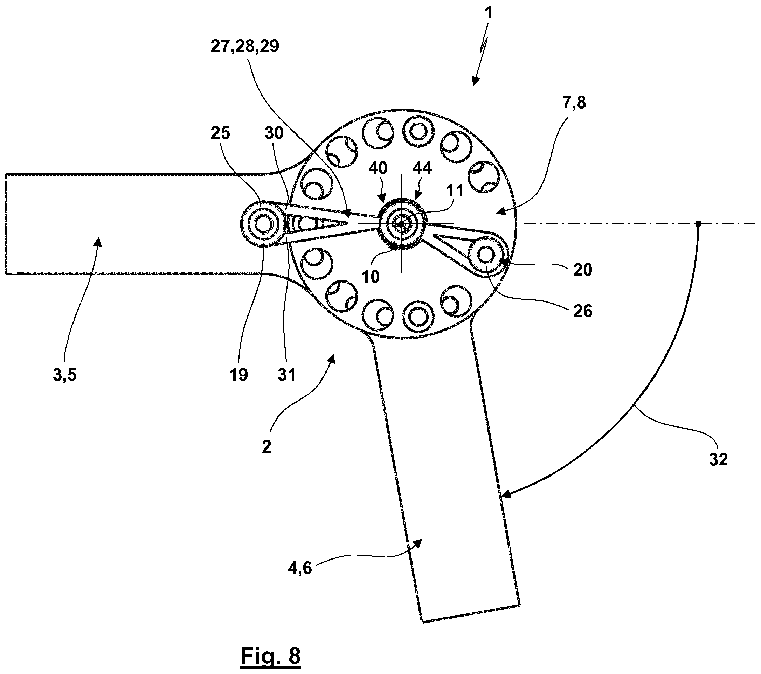

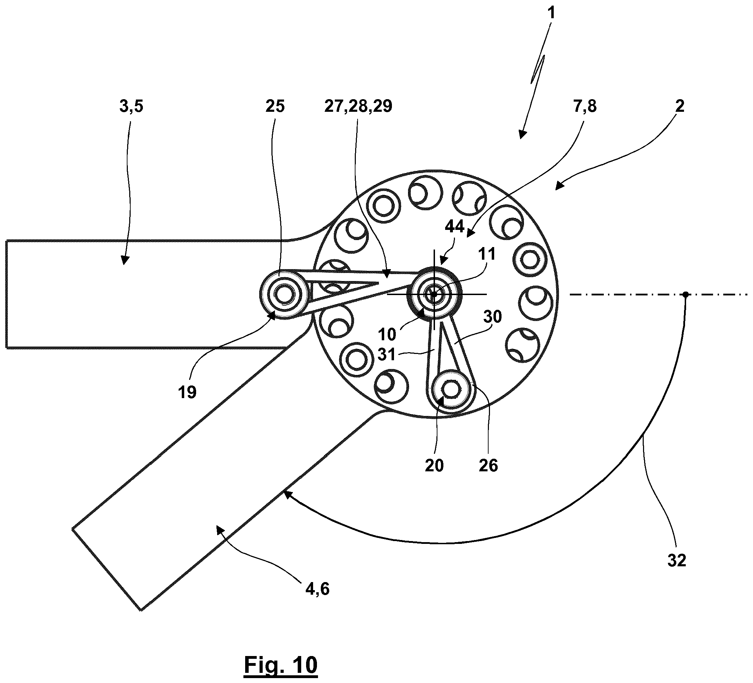

Mit weiterer Erhöhung des Beugewinkels 32 gemäß **Fig. 7** **und** **8** kommen beide Bandteile 30, 31 zur Anlage an die Abstützfläche 33. Die hat zur Folge, dass unabhängig von der Federkraft in der Federeinrichtung 27 die Federeinrichtung 27 kein Quengelmoment erzeugen kann, da die Federkraft der Federeinrichtung 27 keinen Hebelarm hinsichtlich der Schwenkachse 11 der Schienenteile 5, 6 aufweist. Für eine weitere Erhöhung des Beugewinkels 32 ist somit keine Aufbringung eines Moments durch den Träger der Quengelschiene 1 erforderlich. Es ist auf diese Weise dann eine Verschwenkung bis zum maximalen Beugewinkel 32 gemäß **Fig. 10** möglich, wobei der maximale Beugewinkel dadurch vorgegeben ist, dass der Anschlag 14 zur Anlage an den Gegenanschlag 17 kommt (vgl. **Fig. 9****).**

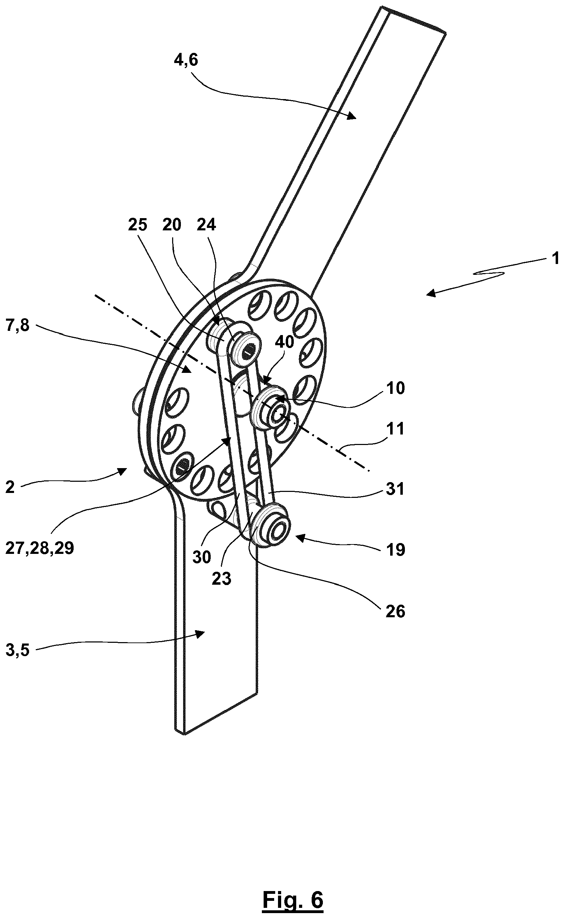

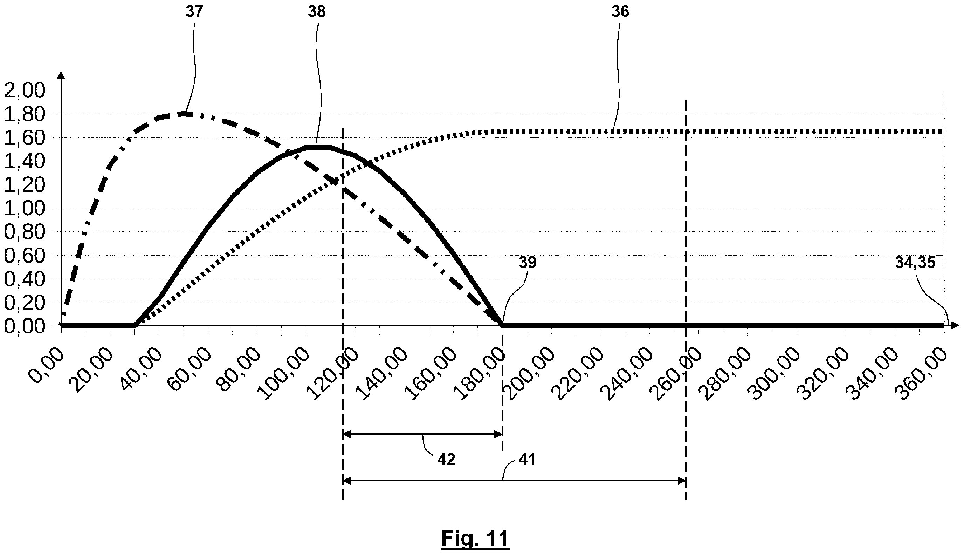

**Fig. 11** zeigt die kinematischen Zusammenhänge für eine Quengelschiene gemäß Fig. 1 bis 10. Hier ist auf der Abszisse 34 der Winkel 35 aufgetragen, unter welchem der Tragbolzen 20 und damit der Federfußpunkt 26 der Federeinrichtung 27 gegenüber einer möglichen Ausgangsstellung, hier eine 9:00-Uhr-Stellung in Fig. 3, angeordnet ist. Die theoretische Variation dieses Winkels 35 in dem dargestellten Winkelbereich von 0° bis 360° für die Darstellung der kinematischen Zusammenhänge erfordert, dass die Anschläge 13, 14 zunächst beseitigt werden. Über dem Winkel 35 sind in Fig. 11 mit dem gepunkteten Verlauf 36 die Federkraft der Federeinrichtung 27, mit dem strichpunktierten Verlauf 37 der Hebelarm einer etwaigen Federkraft der Federeinrichtung 27 und mit dem durchgezogenen Verlauf 38 das sich aus dem Produkt der Federkraft und des Hebelarms ergebende Quengelmoment dargestellt. Der Verlauf 38 des Quengelmoments wird hier auch als "Charakteristik" des Quengelmoments bezeichnet. Auf der Y-Achse ist der Hebelarm in Zentimetern angegeben, während die Federspannung in [100 N] und das Quengelmoment in Nm angegeben sind. Ohne dass eine Einschränkung der Erfindung hierauf erfolgen soll, kann eine erfindungsgemäße Quengelschiene einen Hebelarm, eine Federspannung und ein Quengelmoment entsprechend den dargestellten Verläufen 36, 37, 38 (oder auch mit Verläufen, die gegenüber den dargestellten Verläufen 36, 37, 38 um plus/minus 20 % oder plus/minus 10 % oder 5 % abweichen) aufweisen.

Beträgt der Winkel 35 180°, ist ein Übergang 39 erreicht, für welchen die Federeinrichtung 27 an der Abstützfläche 33 zur Anlage kommt, womit für den Bereich des Winkels 35 von 180° bis 360° der Verlauf des Hebelarms 37 Null beträgt. Hingegen ergibt sich der Verlauf des Hebelarms 37 für den Bereich des Winkels 35 von 0° bis 180° entsprechend den Winkelverhältnissen und den trigonometrischen Funktionen. Die Länge der Federeinrichtung 27 bzw. der Zugfeder 28 ist hier so gewählt, dass die Federeinrichtung 27 im Bereich des Winkels 35 von 0° bis 30° noch nicht gespannt ist, so dass hier der Verlauf 36 Null ist. Erst für einen Winkel 35 größer als 30° wird die Federeinrichtung 27 gespannt. Selbst unter der Annahme einer linearen Federcharakteristik der Federeinrichtung 27 ergibt sich infolge der Winkelverhältnisse im Bereich von 30° bis 180° des Winkels 35 ein nichtlinearer, aber kontinuierlicher Anstieg der Federkraft der Federeinrichtung 27, welche proportional zu dem winkelabhängigen Abstand der beiden Federfußpunkte 25, 26 ist. Liegt für den Winkelbereich von 180° bis 360° die Federeinrichtung 27 an der Abstützfläche 33 an, ändert sich mit der Verschwenkung der Schienenteile 5, 6 der Abstand der Federfußpunkte 25, 26 nicht, so dass hier der Verlauf 36 der Federkraft konstant dem für den Winkel von 180° erreichten Maximum entspricht. Aus dem Produkt der Federkraft gemäß Verlauf 36 mit dem Hebelarm gemäß Verlauf 37 ergibt sich dann der Verlauf des Quengelmoments 38. Angesichts der gewählten Winkelverhältnisse ist hierbei das Quengelmoment bestrebt, in dem Winkelbereich von 30° bis 180° einen Winkel 35 zu reduzieren.

Das Quengelmoment weist an dem Übergang 39 einen Knick auf. Der Knick im Bereich des Übergangs 39 und ein Quengelmoment von Null im Winkelbereich von 180° bis 360° werden hervorgerufen durch einen Umschaltmechanismus 40. Der Umschaltmechanismus 40 besteht darin, dass sich bewegungsgesteuert und in Abhängigkeit von dem Winkel 35 bzw. dem Beugewinkel 32 unterschiedliche Randbedingungen der Federeinrichtung 27 ergeben:
a) Für Winkelbereiche vor dem Erreichen des Übergangs 39 ist die Federeinrichtung 27 mit deren Federfußpunkten 25, 26 ausschließlich einerseits mit dem Schienenteil 5 und andererseits mit dem Schienenteil 6, hier mit der Tragscheibe 8, verbunden, was auch als erste Kopplung 43 bezeichnet wird. Eine Veränderung des Winkels 35 oder des Beugewinkels 32 führt zu einer Veränderung des Abstandes der Federfußpunkte 25, 26, so dass sich winkelabhängig auch eine veränderte Federkraft in der Federeinrichtung 27 ergibt.
b) Kommt hingegen die Federeinrichtung 27 bewegungsgesteuert durch die Veränderung des Winkels 35 bzw. des Beugewinkels 32 zur Anlage an die Abstützfläche 33 der Abstützung 10, erfolgt die bewegungsgesteuerte Umschaltung des Umschaltmechanismus 40. Dies hat zur Folge, dass sich nach der Betätigung des Umschaltmechanismus 40 bzw. nach dem Übergang 39 mit einer Änderung des Winkels 35 im Winkelbereich von 180° bis 360° bzw. mit einer Veränderung des Beugewinkels 32 eine zusätzliche Randbedingung der Federeinrichtung 27 ergibt, nämlich die Anlage derselben an der Abstützfläche 33. Dies wird hier auch als zweite Kopplung 44 bezeichnet. Die Veränderung des Winkels hat dann keine Veränderung des Abstandes der Federfußpunkte 25, 26, keine Veränderung der Dehnung der Federeinrichtung 27 und keine Veränderung der Federkraft 36 zur Folge. Darüber hinaus ist für diesen Winkelbereich der Hebelarm 37 der Federkraft 36 Null, womit auch das Quengelmoment Null beträgt.

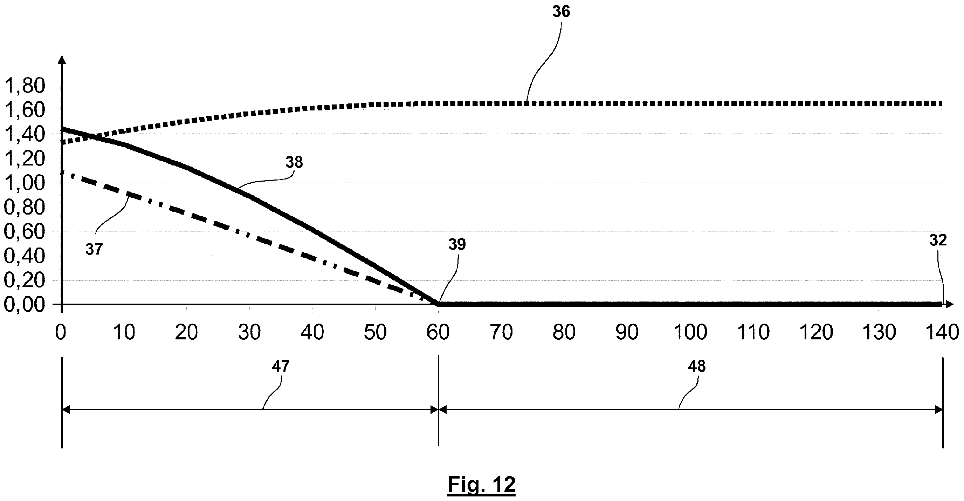

Die Charakteristik des Quengelmoments gemäß Fig. 11 kann je nach Anwendungsbereich unterschiedlich genutzt werden. Hierbei erfolgt eine Einstellung des genutzten Bereichs der Charakteristik durch die Umfangsposition des Tragbolzens 20 und damit des Federfußpunktes 26 in der Streckstellung. Wird beispielsweise der Tragbolzen 20 in der 9:00-Uhr-Stellung in der Streckstellung an der Tragscheibe 8 montiert, führt die Erhöhung des Beugewinkels 32 zum Durchlaufen des Anfangsbereichs der Charakteristik des Quengelmoments gemäß Fig. 11 ausgehend von dem Winkel 35 von Null. Für das in den Fig. 1 bis 10 dargestellte Ausführungsbeispiel entspricht die Streckstellung ungefähr einem Winkel 35 in Fig. 11 von 120° (vgl. den Winkel 35 in Fig. 3), während für den maximalen Beugewinkel 32 gemäß Fig. 10 der Winkel 35 ungefähr 260° entspricht. Der für die Quengelschiene gemäß Fig. 1 bis 10 durchlaufende Bereich der Charakteristik des Quengelmoments ist in Fig. 11 mit dem Bezugszeichen 41 gekennzeichnet. Dieser Ausschnitt der Charakteristik mit der zugeordneten Federkraft und dem Hebelarm ist nochmals in **Fig. 12** dargestellt, wobei hier aber als Abszisse nicht der Winkel 35 gewählt ist, sondern der Beugewinkel 32 gemäß Fig. 1 bis 10. Ein derartiger Bereich des Beugewinkels 32 kann beispielsweise genutzt werden bei Einsatz der Quengelschiene 1 für ein Ellbogengelenk oder ein Kniegelenk. Zu erkennen ist in Fig. 12, dass in einem ersten Schwenkbereich 47 das Quengelmoment erzeugt wird, während in einem zweiten Schwenkbereich 48 kein Quengelmoment erzeugt wird. Der Übergang von dem ersten Schwenkbereich 47 zu dem zweiten Schwenkbereich 48 erfolgt über den Übergang 39.

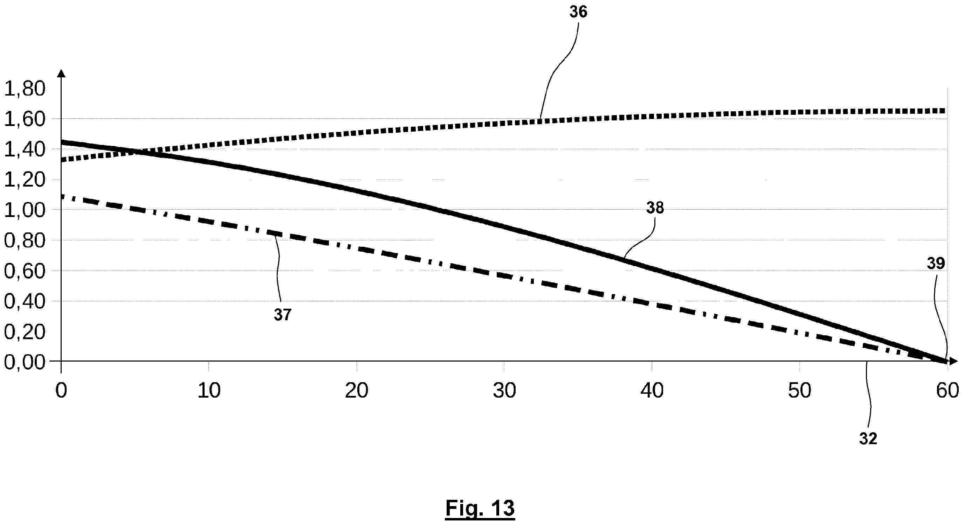

Für einen anderen Einsatzbereich kann durchaus ein anderer Bereich 42 der Charakteristik des Quengelmoments genutzt werden. **Fig. 13** zeigt einen Bereich 42 der Charakteristik des Quengelmoments im Bereich des Winkels 35 von 120° bis 180°, was einem Beugewinkel 32 von 0° bis 60° entspricht. Zu erkennen ist hier, dass nicht zwingend bei dem Einsatz der Quengelschiene 1 der Übergang 39 mit der Betätigung des Umschaltmechanismus 40 genutzt wird. Den Charakteristika des Quengelmoments gemäß Fig. 12 und Fig. 13 ist aber zu entnehmen, dass in der Quengelstellung, welche hier der Streckstellung mit einem Beugewinkel 32 von Null entspricht, das Quengelmoment ein absolutes Maximum hat und mit einer Erhöhung des Beugewinkels 32 das Quengelmoment kontinuierlich kleiner wird, was gemäß Fig. 13 ohne einen Knick auf Null erfolgt, während dies gemäß Fig. 12 für einen Knick im Bereich des Übergangs 39 erfolgt. Es versteht sich, dass ein entsprechender Einsatz derselben Quengelschiene mit derselben Charakteristik möglich ist, wenn hier ein Quengeln für eine Flexion erfolgt, bei welcher die Quengelstellung nicht die Streckstellung ist, sondern eine maximal gebeugte Stellung. Auch in diesem Fall ist das Quengelmoment in der Quengelstellung, hier der maximalen Beugestellung, maximal mit den entsprechenden Verläufen mit einer Verkleinerung des Beugewinkels.

Vor dem Übergang 39 bzw. vor der Betätigung des Umschaltmechanismus 40 besteht eine Kopplung 43 der Federeinrichtung 27 mit den beiden Schienenteilen 5, 6 ausschließlich in Form der Anlenkung der Federfußpunkte 25, 26 an diesen. Hingegen ist nach dem Übergang 39 bzw. nach der Betätigung des Umschaltmechanismus 40 eine Kopplung 44 der Federeinrichtung sowohl mit der Anlenkung der Federfußpunkte 25, 26 an den Schienenteilen 5, 6 als auch mit der Anlage der Federeinrichtung 27 an der Abstützfläche 33 der Abstützung 10 gebildet.

In Fig. 5 ist des Weiteren zu erkennen, dass die Verbindungsachsen 45, 46 einerseits des Tragbolzens 19 oder Federfußpunktes 25 mit der Schwenkachse 11 und andererseits der Schwenkachse 11 mit dem Tragbolzen 20 bzw. dem Federfußpunkt 26 einen Winkel bilden, der kleiner ist als 180°, während die Längsachsen der Schienenteile 5, 6 einen Winkel bilden, der größer ist als 180°. Dies hat zur Folge, dass vor der Betätigung des Umschaltmechanismus 40 das Quengelmoment bestrebt ist, den Beugewinkel 32 zu reduzieren und somit ein Quengelmoment in Richtung der Quengelstellung hervorruft.

Für das in Fig. 1 bis 10 dargestellte Ausführungsbeispiel der Quengelschiene 1 war die Tragscheibe 8 (für eine einmal gewählte Einstellung) fest mit dem Schienenteil 6 verbunden, so dass die Federfußpunkte 25, 26 mit der Veränderung des Beugewinkels 32 jeweils ihre relative Position an den Schienenteilen 5, 6 nicht geändert haben. Der Umschaltmechanismus 40 war hier zwischen den Federfußpunkten 25, 26 durch Ausbildung einer zusätzlichen Kontaktfläche mit der Abstützfläche 33 ausgebildet.

Vorzugsweise liegt das maximale Quengelmoment und/oder das Quengelmoment in der Quengelstellung im Bereich von 2 Nm bis 8 Nm, insbesondere im Bereich von 3 Nm bis 6 Nm.

### BEZUGSZEICHENLISTE

- 1: Quengelschiene
- 2: Gelenk
- 3: Grundkörper
- 4: Grundkörper
- 5: Schienenteil
- 6: Schienenteil
- 7: Tragkörper
- 8: Tragscheibe
- 9: Tellerschraube
- 10: Abstützung
- 11: Schwenkachse
- 12: Bohrung
- 13: Anschlag
- 14: Anschlag
- 15: Schraube
- 16: Schraube
- 17: Gegenanschlag
- 18: Gegenanschlag
- 19: Tragbolzen
- 20: Tragbolzen
- 21: Gewindebohrung
- 22: Exzentrizität, Radius
- 23: Nut
- 24: Nut
- 25: Federfußpunkt
- 26: Federfußpunkt
- 27: Federeinrichtung
- 28: Zugfeder
- 29: elastisches Band
- 30: Bandteil
- 31: Bandteil
- 32: Beugewinkel
- 33: Abstützfläche
- 34: Abszisse
- 35: Winkel
- 36: Verlauf der Federkraft
- 37: Verlauf des Hebelarms
- 38: Verlauf des Quengelmoments
- 39: Übergang
- 40: Umschaltmechanismus
- 41: Bereich des Quengelmoments
- 42: Bereich des Quengelmoments
- 43: Kopplung
- 44: Kopplung
- 45: Verbindungsachse
- 46: Verbindungsachse
- 47: erster Schwenkbereich
- 48: zweiter Schwenkbereich

## Patentansprüche

1. Dynamische Quengelschiene (1) mit
a) zwei Schienenteilen (5, 6), welche über ein Gelenk (2) verschwenkbar um eine Schwenkachse (11) der Schienenteile (5, 6) miteinander verbunden sind, und
b) einer Federeinrichtung (27), deren Federfußpunkte (25, 26) jeweils mit einem der Schienenteile (5, 6) derart gekoppelt sind, dass
ba) eine Verschwenkung der Schienenteile (5, 6) zu einer veränderten Beaufschlagung der Federeinrichtung (27) führt und
bb) die Federeinrichtung (27) ein in Richtung einer Quengelstellung der Schienenteile (5, 6) wirkendes Quengelmoment auf die Schienenteile (5, 6) ausübt, wobei die Quengelstellung eine Stellung mit maximaler Extension ist,
c) wobei die Federeinrichtung (27) so ausgebildet und mit den Schienenteilen (5, 6) gekoppelt ist, dass der Betrag des Quengelmoments mit Annäherung an die Quengelstellung der Schienenteile (5, 6) größer wird und
d) wobei ein bewegungsgesteuert durch die Verschwenkung der Schienenteile (5, 6) betätigter Umschaltmechanismus (40) vorhanden ist, welcher mit seiner Betätigung die Kopplung (43, 44) der Schienenteile (5, 6) mit der Federeinrichtung (27) verändert, und
e) wobei die Quengelstellung der Schienenteile (5, 6) durch einen Anschlag der Quengelschiene (13, 18) vorgegeben ist, und
f) wobei die Federeinrichtung (27) eine Längsfeder ist, deren Federkraft von einer Dehnung in Längsrichtung abhängig ist,
**dadurch gekennzeichnet, dass**
g) der Umschaltmechanismus (40) darin besteht, dass in einem ersten Schwenkbereich (47) der Schienenteile (5, 6) die Längsfeder ausschließlich über die Federfußpunkte (25, 26) der Längsfeder mit den Schienenteilen (5, 6) gekoppelt ist, während in einem zweiten Schwenkbereich (48) der Schienenteile (5, 6) die Längsfeder zusätzlich zwischen den beiden Federfußpunkten (25, 26) an einer Abstützung (10) der Quengelschiene (1) abgestützt ist und durch diese umgelenkt wird.

2. Quengelschiene (1) nach Anspruch 1, **dadurch gekennzeichnet, dass** die Quengelstellung eine Streckstellung der Schienenteile (5, 6) ist und/oder die Ausgangsstellung der Schienenteile (5, 6) eine maximale Beugestellung der Schienenteile (5, 6) ist.

3. Quengelschiene (1) nach Anspruch 1 oder 2, **dadurch gekennzeichnet, dass** die Federeinrichtung (27) so ausgebildet und mit den Schienenteilen (5, 6) gekoppelt ist, dass die Charakteristik des Quengelmoments in Abhängigkeit von dem Beugewinkel (32) einen Knick oder einen Sprung aufweist.

4. Quengelschiene (1) nach einem der vorhergehenden Ansprüche, **dadurch gekennzeichnet, dass**
a) der erste Schwenkbereich (47) der Quengelstellung benachbart ist oder diese einschließt und in dem ersten Schwenkbereich (5, 6) die Schienenteile (5, 6) über eine erste Kopplung (43) mit der Federeinrichtung (27) gekoppelt sind, und
b) der zweite Schwenkbereich (48) der Ausgangsstellung benachbart ist oder diese einschließt und in dem zweiten Schwenkbereich (48) die Schienenteile (5, 6) über eine zweite Kopplung (44) mit der Federeinrichtung (27) gekoppelt sind, wobei die zweite Kopplung (44) von der ersten Kopplung (43) abweicht,
c) wobei ein Übergang (39) von dem ersten Schwenkbereich (47) zu dem zweiten Schwenkbereich (48) über die durch die Verschwenkung der Schienenteile (5, 6) bewegungsgesteuerte Betätigung des Umschaltmechanismus (40) erfolgt.

5. Quengelschiene (1) nach Anspruch 4, **dadurch gekennzeichnet, dass** in dem zweiten Schwenkbereich (48) der Betrag des Quengelmoments konstant ist, Null beträgt und/oder maximal 30% des Mittelwerts des Betrags des Quengelmoments im ersten Schwenkbereich (47) beträgt.

6. Quengelschiene (1) nach Anspruch 4 oder 5, **dadurch gekennzeichnet, dass** in dem ersten Schwenkbereich (47) das Quengelmoment
a) für eine Verschwenkung in Richtung der Quengelstellung größer wird und/oder
b) eine Wirkrichtung aufweist, welche bestrebt ist, die Schienenteile (5, 6) in Richtung der Quengelstellung zu verschwenken.

7. Quengelschiene (1) nach einem der vorhergehenden Ansprüche, **dadurch gekennzeichnet, dass** die Charakteristik (38) des Quengelmoments, die Federeinrichtung (27) und/oder die Kopplung (43, 44) der Federeinrichtung (27) mit den Schienenteilen (5, 6) einstellbar ist.

8. Quengelschiene (1) nach einem der vorhergehenden Ansprüche, **dadurch gekennzeichnet, dass**
a) Federfußpunkte (25, 26) der Federeinrichtung (27) jeweils mit einer Exzentrizität (22) gegenüber der Schwenkachse (11) der Schienenteile (5, 6) mit einem Schienenteil (5; 6) gekoppelt sind,
b) wobei die Schienenteile (5, 6) in einer Stellung der Quengelschiene (1) einen Winkel bilden, der größer ist als 180°, während der Winkel der Verbindungsachsen (45, 46) der Federfußpunkte (25, 26) mit der Schwenkachse (11) kleiner ist als 180°.

9. Quengelschiene (1) nach einem der vorhergehenden Ansprüche, **dadurch gekennzeichnet, dass** die zusätzliche Abstützung der Längsfeder im Bereich der Schwenkachse (11) der Schienenteile (5, 6) angeordnet ist.

10. Quengelschiene (1) nach einem der vorhergehenden Ansprüche, **dadurch gekennzeichnet, dass** die Schienenteile (5, 6) in Plattenbauweise ausgebildet sind.

## Claims

1. Dynamic biasing splint (1) comprising
a) two splint parts (5, 6) connected to each other by a joint (2) for being pivoted about a pivot axis (11) of the splint parts (5, 6) and
b) a spring device (27), the spring bases (25, 26) of the spring device (27) each being coupled to one of the splint parts (5, 6) in a way such that
ba) a pivoting of the splint parts (5, 6) leads to a changed bias of the spring device (27) and
bb) the spring device (27) applies a biasing moment on the splint parts (5, 6) acting towards a biasing position of the splint parts (5, 6), the biasing position being a position with a maximum extension,
c) the spring device (27) being designed such that and being coupled to the splint parts (5, 6) such that the absolute value of the biasing moment increases when approaching the biasing position of the splint parts (5, 6) and
d) a switching mechanism (40) being provided which is actuated by motion by the pivoting movement of the splint parts (5, 6) and which when actuated changes the coupling (43, 44) of the splint parts (5, 6) to the spring device (27) and
e) the biasing position of the splint parts (5, 6) being defined by a stop of the biasing splint (13, 18) and
f) the spring device (27) being a longitudinal spring, the spring force depending on an elongation in longitudinal direction,
**characterised in that**
g) the switching mechanism (40) is provided **in that** in a first pivoting region (47) of the splint parts (5, 6) the longitudinal spring is exclusively coupled via the spring bases (25, 26) of the longitudinal spring to the splint parts (5, 6) whereas in a second pivoting region (48) of the splint parts (5, 6) the longitudinal spring is additionally supported between the two spring bases (25, 26) on a support (10) of the biasing splint (1) and deflected by the support (10).

2. Biasing splint (1) of claim 1, **characterised in that** the biasing position is a stretched position of the splint parts (5, 6) and/or the starting position of the splint parts (5, 6) is a maximally bent position of the splint parts (5, 6).

3. Biasing splint (1) of claim 1 or 2, **characterised in that** the spring device (27) is designed such that and coupled to the splint parts (5, 6) such that the characteristic of the biasing moment as a function of the deflection angle (32) comprises a jump or kink.

4. Biasing splint (1) of one of the preceding claims, **characterised in that**
a) the first pivoting region (47) is located adjacent to the biasing position or includes the biasing position and in the first pivoting region (5, 6) the splint parts (5, 6) are coupled by a first coupling (43) to the spring device (27) and
b) the second pivoting region (48) is located adjacent to the starting position or includes the starting position and in the second pivoting region (48) the splint parts (5, 6) are coupled via a second coupling (44) to the spring device (27), the second coupling (44) differing from the first coupling (43),
c) a transition (39) from the first pivoting region (47) to the second pivoting region (48) is provided by an actuation of the switching mechanism (40) controlled by motion when pivoting the splint parts (5, 6).

5. Biasing splint (1) of claim 4, **characterised in that** in the second pivoting region (48) the absolute value of the biasing moment is constant, equals zero and/or is at the most 30 % of the mean value of the absolute value of the biasing moment in the first pivoting region (47).

6. Biasing splint (1) of claim 4 or 5, **characterised in that** in the first pivoting region (47) the biasing moment
a) increases for a pivoting movement towards the biasing position and/or
b) is effective in a direction which intends to cause a pivoting the splint parts (5, 6) towards the biasing position.

7. Biasing splint (1) of one of the preceding claims, **characterised in that** it is possible to adjust the characteristic (38) of the biasing moment, the spring device (27) and/or the coupling (43, 44) of the spring device (27) to the splint parts (5, 6).

8. Biasing splint (1) of one of the preceding claims, **characterised in that**
a) spring bases (25, 26) of the spring device (27) are each coupled with an eccentricity relative to the pivot axis (11) of the splint parts (5, 6) to a splint part (5; 6),
b) the splint parts (5, 6) in one position of the biasing splint (1) forming an angle being larger than 180° whereas the angle of the connecting axes (45, 46) of the spring bases (25, 26) and the pivot axis (11) is smaller than 180°.

9. Biasing splint (1) of one of the preceding claims, **characterised in that** the additional support of the longitudinal spring is arranged in the region of the pivot axis (11) of the splint parts (5, 6).

10. Biasing splint (1) of one of the preceding claims, **characterised in that** the splint parts (5, 6) have a sheet construction.

## Revendications

1. Glissière de correction (1) avec
a) deux parties de glissière (5, 6) qui sont reliées entre elles par l'intermédiaire d'une articulation (2) de manière pivotante autour d'un axe de pivotement (11) des parties de glissière (5, 6) et
b) un dispositif à ressort (27), dont les pieds de ressort (25, 26) sont couplés chacun avec une des parties de glissière (5, 6) de façon à ce que
ba) un pivotement des parties de glissière (5, 6) conduit à une modification de la sollicitation du dispositif à ressort (27) et
bb) le dispositif à ressort (27) exerce un couple de correction agissant sur les parties de glissière (5, 6) en direction d'une position de correction des parties de glissière (5, 6), dans lequel la position de correction est une position avec extension maximale,
c) dans laquelle le dispositif à ressort (27) est conçu et couplé avec les parties de glissière (5, 6) de façon à ce que la valeur du couple de correction augmente lorsqu'on s'approche de la position de correction des parties de glissière (5, 6) et
d) dans laquelle un mécanisme de commutation (40) actionné, de manière contrôlée par le mouvement, par le pivotement des parties de glissière (5, 6), est présent, qui modifie, avec son actionnement, le couplage (43, 44) des parties de glissière (5, 6) avec le dispositif à ressort (27) et
e) dans laquelle la position de correction des parties e glissière (5, 6) est prédéterminée par une butée de la glissière de correction (13, 18) et
f) le dispositif à ressort (27) est un ressort longitudinal dont la force élastique dépend d'une dilatation dans la direction longitudinale,
**caractérisée en ce que**
g) le mécanisme de commutation (40) consiste **en ce que**, dans une première zone de pivotement (47) des parties de glissière (5, 6), le ressort longitudinal est couplé exclusivement par l'intermédiaire des pieds du ressort (25, 26) du ressort longitudinal avec les parties de glissière (5, 6), tandis que, dans une deuxième zone de pivotement (48) des parties de glissière (5, 6), le ressort longitudinal s'appuie, en outre, entre les deux pieds de ressort (25, 26), contre un appui (10) de la glissière de correction (1) et est dévié par celui-ci.

2. Glissière de correction (1) selon la revendication 1, **caractérisée en ce que** la position de correction est une position d'extension des parties de glissière (5, 6) et/ou la position de départ des parties de glissière est une position de flexion maximale des parties de glissière (5, 6).

3. Glissière de correction (1) selon la revendication 1 ou 2, **caractérisée en ce que** le dispositif à ressort (27) est conçu et couplé avec les parties de glissière (5, 6) de façon à ce que la courbe caractéristique du couple de correction en fonction de l'angle de flexion (32) présente un coude ou un saut.

4. Glissière de correction (1) selon l'une des revendications précédentes, **caractérisée en ce que**
a) la première zone de pivotement (47) est adjacente à la position de correction ou inclut celle-ci et, dans la première zone de pivotement (5, 6), les parties de glissière (5, 6) sont couplées par l'intermédiaire d'un premier couplage (43) avec le dispositif à ressort (27) et
b) la deuxième zone de pivotement (48) est adjacente à la position de départ ou inclut celle-ci et, dans la deuxième zone de pivotement (48), les parties de glissière (5, 6) sont couplées par l'intermédiaire d'un deuxième couplage (44) avec le dispositif à ressort (27), dans laquelle le deuxième couplage (44) est différent du premier couplage (43),
c) dans laquelle une transition (38) de la première zone de pivotement (47) vers la deuxième zone de pivotement (48) a lieu grâce à l'actionnement, de manière contrôlée par le mouvement, par le pivotement des parties de glissière (5, 6), du mécanisme de commutation (40).

5. Glissière de correction (1) selon la revendication 4, **caractérisée en ce que**, dans la deuxième zone de pivotement (48), la valeur du couple de correction est constante, égale à zéro et/ou représente au maximum 30 % de la valeur moyenne du couple de correction dans la première zone de pivotement (47).

6. Glissière de correction (1) selon la revendication 4 ou 5, **caractérisée en ce que**, dans la première zone de pivotement (47), le couple de correction
a) augmente pour un pivotement en direction de la position de correction et/ou
b) présente une direction d'action qui vise à faire pivoter les parties de glissière (5, 6) en direction de la position de direction.

7. Glissière de correction (1) selon l'une des revendications précédentes, **caractérisée en ce que** la courbe caractéristique (38) du couple de correction, le dispositif à ressort (27) et/ou le couplage (43, 44) du dispositif à ressort (27) peuvent être réglés avec les parties de glissière (5, 6).

8. Glissière de correction (1) selon l'une des revendications précédentes, **caractérisée en ce que**
a) les pieds de ressort (25, 26) du dispositif à ressort (27) sont couplés chacun, avec une excentricité (22) par rapport à l'axe de pivotement (11) des parties de glissière (5, 6), avec une partie de glissière (5, 6),
b) dans laquelle les parties de glissière (5, 6) forment, dans une position de la glissière de correction (1), un angle qui est supérieur à 180°, tandis que l'angle des axes de liaison (45, 46) des pieds de ressort (25, 26) avec l'axe de pivotement (11) est inférieur à 180°.

9. Glissière de correction (1) selon l'une des revendications précédentes, **caractérisée en ce que** l'appui supplémentaire du ressort longitudinal est disposé au niveau de l'axe de pivotement (11) des parties de glissière (5, 6).

10. Glissière de correction (1) selon l'une des revendications précédentes, **caractérisée en ce que** les parties de glissière (5, 6) sont réalisées sous la forme de plaques.
